(19) Europäisches Patentamt / European Patent Office / Office européen des brevets

(11) **EP 3 165 160 B1**

(12) **FASCICULE DE BREVET EUROPEEN**

(45) Date de publication et mention de la délivrance du brevet:
**13.03.2019 Bulletin 2019/11**

(51) Int Cl.:
*A61B 5/04* (2006.01)    *A61B 5/0452* (2006.01)
*A61N 1/37* (2006.01)    *A61N 1/39* (2006.01)
*A61N 1/368* (2006.01)

(21) Numéro de dépôt: **16197131.2**

(22) Date de dépôt: **03.11.2016**

(54) **DISPOSITIF MÉDICAL IMPLANTABLE ACTIF COMPRENANT DES MOYENS DE DÉTECTIONS ET DE QUANTIFICATION DES SITUATIONS DE FUSION**

AKTIVE IMPLANTIERBARE MEDIZINISCHE VORRICHTUNG, DIE MITTEL FÜR DIE ERFASSUNG UND QUANTIFIZIERUNG VON FUSIONSSITUATIONEN UMFASST

ACTIVE IMPLANTABLE MEDICAL DEVICE COMPRISING MEANS FOR DETECTING AND QUANTIFYING FUSION SITUATIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.11.2015 FR 1560620**

(43) Date de publication de la demande:
**10.05.2017 Bulletin 2017/19**

(73) Titulaire: **Sorin CRM SAS**
**92140 Clamart Cedex (FR)**

(72) Inventeurs:
• **MILPIED, Paola**
**75014 Paris (FR)**
• **FEUERSTEIN, Delphine**
**92130 Issy les Moulineaux (FR)**

(74) Mandataire: **Grünecker Patent- und Rechtsanwälte**
**PartG mbB**
**Leopoldstraße 4**
**80802 München (DE)**

(56) Documents cités:
**EP-A1- 2 324 885        EP-A1- 2 368 493**
**EP-A1- 2 742 971        EP-A1- 2 742 973**
**EP-A1- 2 756 865        EP-A1- 2 873 436**
**US-A1- 2012 310 297**

• **MILPIED P ET AL: "Arrhythmia Discrimination in Implantable Cardioverter Defibrillators Using Support Vector Machines Applied to a New Representation of Electrograms", IEEE TRANSACTIONS ON BIOMEDICAL ENGINEERING, IEEE SERVICE CENTER, PISCATAWAY, NJ, USA, vol. 58, no. 6, 1 juin 2011 (2011-06-01), pages 1797-1803, XP011408407, ISSN: 0018-9294, DOI: 10.1109/TBME.2011.2117424**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. L'invention concerne plus particulièrement la situation dite de "fusion", c'est-à-dire la survenue d'une stimulation appliquée de façon plus ou moins concomitante à une dépolarisation spontanée, produite naturellement par le noeud sinusal (dans le cas de fusion atriale) et/ou par le noeud atrioventriculaire (dans le cas de fusion ventriculaire).

Dans le cadre de la présente invention, le terme de "rythme spontané" sera entendu comme une dépolarisation spontanée dans les ventricules empruntant les voies de conduction naturelles (faisceau de His, fibres de Purkinje, etc.), quelle que soit l'origine de la dépolarisation dans les oreillettes (stimulée ou spontanée).

La situation peut se présenter en particulier dans le contexte d'un test dit "test de capture" consistant, après avoir stimulé une cavité, à détecter dans cette cavité la présence ou non d'une "onde évoquée", c'est-à-dire d'une onde de dépolarisation induite par la stimulation, ceci afin de déterminer si cette stimulation a été efficace ou non. Ce test est notamment utilisé pour modifier à intervalles réguliers l'amplitude ou la largeur des impulsions appliquées au site de stimulation, afin d'adapter l'énergie délivrée en fonction du seuil d'efficacité ou "seuil d'entrainement" de ce site, afin que l'énergie soit suffisante pour induire de façon certaine une onde évoquée, mais sans être excessive de manière à ne pas obérer la durée de vie du dispositif.

Si une fusion intervient au cours de ce test, c'est-à-dire si une stimulation est appliquée au moment où survient un événement QRS spontané, l'algorithme de test va se trouver leurré par cette situation car la morphologie de l'onde évoquée sera différente. De fait, il convient de pouvoir détecter une situation de suspicion de fusion, afin tout d'abord d'invalider le test puis d'adapter les paramètres de stimulation (raccourcissement du délai atrioventriculaire DAV ou augmentation de la fréquence de stimulation) afin de s'affranchir de ces erreurs pour mener à bien le test de seuil de capture.

De façon générale, il est important de pouvoir détecter la présence d'une fusion dans le cadre d'une stimulation antibradycardique d'un dispositif fonctionnant en stimulation "double chambre" classique, c'est-à-dire où le dispositif surveille l'activité ventriculaire après un événement auriculaire spontané (onde P) ou stimulé (impulsion A) et déclenche une stimulation du ventricule droit (impulsion V) si aucune activité spontanée ventriculaire (onde R) n'a été détectée à l'issue d'un délai dit "délai atrioventriculaire" (DAV ou AVD). Dans ce contexte, la présence d'une fusion peut témoigner d'une conduction spontanée préservée, et donc de la non-nécessité de stimulation : si l'on inhibe la stimulation, par exemple en allongeant le DAV en cas de fusion ventriculaire, non seulement il n'y aura pas d'effet délétère de la stimulation (qui pourrait sinon provoquer notamment une désynchronisation et conduire à une fibrillation atriale ou à une aggravation de l'insuffisance cardiaque à long terme), mais également on préserve l'autonomie du dispositif par une moindre sollicitation du générateur d'impulsions.

Une autre configuration où il est utile de pouvoir détecter une situation de fusion est celle d'une thérapie de resynchronisation, dite "CRT" (*Cardiac Resynchronization Therapy*) ou "BVP" (*Bi-Ventricular Pacing*), consistant à surveiller en continu le rythme cardiaque et délivrer si nécessaire au coeur des impulsions électriques permettant de provoquer une contraction conjointe et permanente des ventricules gauche et droit afin de resyn-chroniser ces derniers. Une telle thérapie CRT permet d'optimiser le cycle contraction/relaxation avec un bénéfice direct facilitant le travail du coeur, notamment dans les pathologies d'insuffisance cardiaque où l'on cherche à stabiliser le phénomène de remodelage cardiaque (c'est-à-dire l'ensemble des modifications du coeur engendrées en réponse à une pathologie, et qui est généralement associé à un plus mauvais pronostic), et même de contrer ce phénomène ("remodelage inverse"), avec pour le patient un meilleur pronostic.

La thérapie CRT peut être mise en oeuvre avec stimulation du seul ventricule gauche, lorsque la conduction atrioventriculaire droite native est préservée, réduisant d'autant le besoin d'une stimulation droite, inutile et délétère. Il convient alors de chercher à favoriser la fusion de cette stimulation gauche avec la conduction spontanée du ventricule droit. En présence d'une capture complète, ou si la stimulation engendre une dépolarisation trop proche de la capture complète, le DAV devra être allongé pour garantir la fusion et obtenir l'effet mécanique souhaité de resynchronisation des deux ventricules.

Ce cas particulier d'une thérapie CRT avec stimulation du seul ventricule gauche n'est cependant pas limitatif, le fait de favoriser la fusion dans le cadre d'une thérapie CRT pouvant également s'appliquer à de la stimulation multipoints et/ou biventriculaire. La fusion peut être observée sur l'électrocardiogramme (ECG) de surface, de préférence en utilisant un grand nombre de dérivations pour visualiser les moindres changements morphologiques des ondes d'activation en cas de fusion. Toutefois, l'appréciation de la fusion par l'ECG est en général qualitative, et variable selon l'opérateur. En tout état de cause, une détermination automatique de la fusion, avec des moyens de quantification objective et établissement d'une métrique ou d'une échelle (ci-après "degré de fusion"), est nécessaire pour améliorer la resynchronisation ventriculaire et donc pour un fonctionnement optimal des dispositifs CRT implantables.

Le EP 2 324 885 A1 (Sorin CRM) décrit une technique de classification de chaque cycle cardiaque en i) cycles en capture complète, ii) cycles en fusion ou iii) cycles avec perte de capture. Cette classification est opérée par comparaison du cycle courant avec une référence unique mémorisée, correspondant à une situation de capture complète. Pour différencier une situation de fusion d'une situation de perte de capture, ce document propose une méthode basée sur l'analyse bidimensionnelle de deux signaux d'électrogramme endocavitaire (EGM) recueillis concurremment sur deux voies distinctes et provenant de la même cavité, notamment du ventricule droit.

Les deux voies EGM différentes peuvent être en particulier celles d'un signal unipolaire (signal lointain recueilli entre le boitier et une électrode distale ou bien proximale) et celle d'un signal bipolaire (signal proche recueilli entre une électrode distale et une électrode proximale), respectivement. L'analyse bidimensionnelle est effectuée sur une "boucle cardiaque" ou "vectogramme" (VGM), qui est la représentation dans un espace à deux dimensions de l'un des deux signaux par rapport à l'autre, chaque battement cardiaque ou fraction significative de battement étant alors représenté par son vectogramme dans le plan ainsi défini.

Plus précisément, le procédé d'analyse consiste à décrire chaque VGM par le vecteur vitesse en chaque point de la boucle. Le VGM produit pour une énergie de stimulation donnée est ensuite comparé à un VGM de référence obtenu à énergie maximale.

Pour différencier la fusion de la perte de capture, l'algorithme se base sur les valeurs du descripteur utilisé pour la comparaison : ce descripteur est comparé à un premier seuil, qui permet de décider une suspicion de fusion (vs. capture), puis à un deuxième seuil, correspondant à une perte de capture.

Cette technique présente l'inconvénient de fonctionner subsidiairement et en "tout-ou-rien" pour ce qui est de la fusion : c'est-à-dire que tout ce qui ne ressemble ni à une capture complète ou partielle, ni à une perte de capture est considéré, à défaut, comme une situation de fusion. En d'autres termes, il s'agit d'une technique de classification, binaire. Il ne s'agit pas d'une technique de quantification de la fusion qui permettrait d'évaluer sur une échelle le décalage temporel plus ou moins important entre les ondes de dépolarisation stimulée et spontanée (si cette dernière est présente) correspondant respectivement à l'application de la stimulation (onde évoquée) et à la survenue du complexe QRS spontané.

De plus, cette méthode produit un résultat convenable pour suspecter une situation de fusion lorsque les DAV sont courts, mais n'est pas très discriminante sur la gamme des DAV longs. En particulier, la distinction entre fusion et pseudo-fusion (cas où la dépolarisation est totalement spontanée en présence d'une stimulation qui est appliquée trop tardivement) ne peut pas être mise en évidence de façon fiable.

Le EP 2 756 865 A1 décrit une autre technique permettant non seulement de déterminer les situations de fusion, mais en plus de quantifier une fusion. Cette technique repose sur l'analyse d'un EGM unipolaire avec comparaison, pour chaque cycle cardiaque, de l'EGM enregistré avec deux signaux de référence, pour produire deux indices de corrélation adaptatifs signés (ASCI), compris entre -1 et +1. Ces indices sont ensuite multipliés pour traduire la ressemblance plus ou moins grande du cycle cardiaque aux références enregistrées, une situation intermédiaire correspondant à une fusion, quantifiée par le produit des deux indices calculés pour le cycle cardiaque.

Toutefois, cette technique ne permet pas toujours de mettre en évidence la fusion, le signal sur la voie unipolaire ne reflétant pas toujours suffisamment certains changements caractéristiques de la morphologie ou du synchronisme temporel produits par une fusion.

Le but de l'invention est de remédier à ces inconvénients précités, en proposant un perfectionnement à la méthode du EP 2 324 885 A1 précité, qui permette non seulement de discriminer en toute fiabilité des situations effectives de fusion, mais également de quantifier le degré de fusion d'une manière qui reflète de façon exacte l'activation électrique résultant de la concomitance de deux fronts d'onde, l'un issu de la dépolarisation spontanée et l'autre induit par la stimulation.

L'invention est applicable à une large variété de dispositifs, aussi bien à des dispositifs de stimulation antibradycardique conventionnelle (où une fusion est généralement délétère, et inappropriée dans le cas d'un test de capture) qu'à des dispositifs de resynchronisation CRT (où l'on peut notamment chercher à optimiser une fusion entre une stimulation du ventricule gauche et une contraction spontanée du ventricule droit, ou *vice versa*).

Par ailleurs, dans la présente demande on décrira principalement une fusion par rapport à des stimulations et détections ventriculaires, mais on notera que la présente invention peut être également appliquée à des stimulations et détections auriculaires, pour déterminer une fusion au niveau de l'oreillette (dans ce cas ce n'est pas le DAV qui variera, mais la fréquence de stimulation).

Plus précisément, l'invention propose un dispositif comprenant, de manière en elle-même connue d'après le EP 2 324 885 A1 précité :

- des moyens de stimulation contrôlée du coeur ;
- des moyens de recueil de signaux de dépolarisation ventriculaire et/ou auriculaire, aptes à recueillir au cours d'un même cycle cardiaque, concurremment sur des voies respectives distinctes, au moins deux signaux différents d'électrogramme endocavitaire, EGM, et en dériver au moins deux composantes temporelles distinctes respectives ;

et

- des moyens d'analyse du cycle cardiaque courant, comprenant :

  des moyens aptes à combiner les au moins deux composantes temporelles en au moins une caractéristique 2D paramétrique non-temporelle, paramétrée par le temps, représentative dudit cycle cardiaque, à partir des variations de l'une des composantes temporelles en fonction de l'autre ; et
  des moyens aptes à opérer une comparaison de la caractéristique 2D du cycle courant avec au moins une caractéristique 2D de référence antérieure mémorisée par le dispositif, pour en dériver une valeur d'au moins un descripteur représentatif de la similarité entre la caractéristique 2D du cycle courant et la caractéristique 2D de référence.

De façon caractéristique de l'invention, ce dispositif comprend des moyens de quantification d'un degré de fusion, comportant :

- des moyens pour opérer une première comparaison, de la caractéristique 2D du cycle courant avec une première caractéristique 2D de référence en situation de capture complète mémorisée par le dispositif, pour en dériver une première valeur dudit au moins un descripteur ;
- des moyens pour opérer une seconde comparaison, de la caractéristique 2D du cycle courant avec une seconde caractéristique 2D de référence (Référence n°2) en situation de rythme spontané ventriculaire mémorisée par le dispositif, pour en dériver une seconde valeur dudit au moins un descripteur ; et
- des moyens pour dériver une métrique quantifiant, par une troisième valeur, un taux de fusion en cas de survenue d'une situation de fusion, ladite troisième valeur étant fonction à la fois de ladite première valeur et de ladite seconde valeur, et
  ladite troisième valeur étant une valeur intermédiaire entre deux extrema respectifs correspondant auxdites première et seconde valeurs obtenues respectivement en situation de capture complète et en situation de rythme spontané.

Dans un mode de réalisation préférentiel, ledit au moins un descripteur comprend deux descripteurs différents, définissant un espace bidimensionnel dont les deux dimensions correspondent aux deux descripteurs. Lesdites première et seconde valeurs sont alors représentées par deux points respectifs de cet espace bidimensionnel, et le dispositif comprend en outre des moyens de calcul de ladite troisième valeur en fonction des distances respectives séparant lesdits deux points d'un repère d'origine de l'espace bidimensionnel.

L'un des deux descripteurs différents peut notamment être le coefficient de corrélation entre les normes des vecteurs vitesse respectifs de la caractéristique 2D courante et de la première ou seconde caractéristique 2D de référence, l'autre des deux descripteurs étant l'angle moyen entre les vecteurs vitesse respectifs de la caractéristique 2D courante et de la première ou seconde caractéristique 2D de référence.

De préférence, le taux de fusion, exprimé en termes de pourcentage de capture complète, est donné pour un cycle courant en fusion plus proche de la capture que du rythme spontané par :

$$50 + [50*(A2-A1)/(X2-X1)]$$

et pour un cycle courant en fusion plus proche du rythme spontané que de la capture par :

$$50 - [50 * (B1-B2)/(Y1-Y2)].$$

A2-A1 étant l'écart des distances A1 et A2 séparant, dans ledit espace bidimensionnel, des points représentant respectivement lesdites première et seconde valeurs pour ledit cycle courant en fusion plus proche de la capture que du rythme spontané,

B1-B2 étant l'écart des distances B1 et B2, séparant, dans ledit espace bidimensionnel, des points (P1, P2) représentant respectivement lesdites première et seconde valeurs pour ledit cycle courant en fusion plus proche du rythme spontané que de la capture,

X2-X1 étant l'écart des distances X1 et X2 séparant, dans ledit espace bidimensionnel, des points représentant respectivement lesdites première et seconde valeurs pour des cycles en capture complète, et

Y1-Y2 étant l'écart des distances Y1 et Y2 séparant, dans ledit espace bidimensionnel, des points représentant respectivement lesdites première et seconde valeurs pour des cycles en rythme spontané.

Les moyens d'établissement de ladite première caractéristique 2D de référence comprennent avantageusement :

- des moyens d'application d'une stimulation avec un délai atrioventriculaire court et une fréquence de stimulation supérieure au rythme sinusal spontané, de manière à obtenir une série de cycles cardiaques en situation de capture complète ; et
- des moyens de sélection d'un cycle cardiaque représentatif à partir desdits cycles cardiaques, et de détermination à partir de ce cycle représentatif de ladite première caractéristique 2D de référence correspondante.

Les moyens d'établissement de ladite seconde caractéristique 2D de référence comprennent avantageusement :

- des moyens d'application d'une stimulation avec un délai atrioventriculaire long et une fréquence de stimulation supérieure au rythme sinusal spontané, de manière à obtenir une série de cycles cardiaques en situation de rythme ventriculaire spontané ; et
- des moyens de sélection d'un cycle cardiaque représentatif à partir desdits cycles cardiaques, et de détermination à partir de ce cycle représentatif de ladite seconde caractéristique 2D de référence correspondante.
- De préférence, il est en outre prévu des moyens de validation desdites caractéristiques 2D de référence, comprenant :
- des moyens pour opérer une comparaison de ladite première caractéristique 2D de référence avec ladite seconde caractéristique 2D de référence, pour en dériver au moins un descripteur représentatif de la similarité entre la première et la seconde caractéristique 2D de référence ; et
- des moyens pour valider ou invalider lesdites caractéristiques 2D de référence selon que ledit au moins un descripteur vérifie(nt) ou non au moins un critère de validation prédéterminé.

En particulier, il peut être alors en outre prévu :

- des moyens d'application d'une stimulation avec un délai atrioventriculaire long et une fréquence de stimulation inférieure au rythme sinusal spontané, de manière à obtenir une série de cycles cardiaques en situation de dépolarisation spontanée à la fois auriculaire et ventriculaire ;
- des moyens de sélection d'un cycle cardiaque représentatif à partir desdits cycles cardiaques, et de détermination à partir de ce cycle représentatif d'une troisième caractéristique 2D de référence correspondante ; et
- des moyens de détermination, à partir desdites seconde et troisième caractéristiques 2D de référence, d'une valeur de décalage entre évènement auriculaire stimulé et événement auriculaire spontané.

On va maintenant décrire un exemple de mise en oeuvre de la présente invention, en référence aux dessins annexés où les mêmes références désignent d'une figure à l'autre des éléments identiques ou fonctionnellement semblables.

La Figure 1 est une vue générale montrant un dispositif de stimulation antibradycardique de type "double chambre", avec son générateur et une sonde implantée dans le coeur droit.
La Figure 2 est une vue générale montrant un dispositif de stimulation CRT avec son générateur et des sondes cardiaques droite et gauche implantées dans le coeur.
La Figure 3 est un exemple de signaux EGM obtenus sur des voies respectivement ventriculaire bipolaire et ventriculaire unipolaire de l'une des sondes de la Figure 1.
La Figure 4 illustre des exemples de signaux recueillis sur une voie bipolaire et sur une voie unipolaire, pour différentes situations correspondant à une capture ventriculaire complète, à une perte totale de capture laissant apparaitre le rythme ventriculaire spontané, et pour un certain nombre de cycles intermédiaires à tester, avec présence éventuelle d'une fusion plus ou moins marquée, pour des DAV intermédiaires entre les deux situations de capture et de perte de capture.
La Figure 5 illustre la manière de combiner entre eux les signaux bipolaire et unipolaire recueillis dans une même cavité ventriculaire pour construire une caractéristique bidimensionnelle de type vectogramme.
La Figure 6 est un exemple de vectogramme échantillonné obtenu pour un cycle cardiaque échantillonné à 128 Hz, avec représentation des vecteurs vitesse en divers points successifs.
La Figure 7 est un schéma de principe illustrant la mise en oeuvre de l'invention.
La Figure 8 est un organigramme simplifié présentant la séquence d'étapes exécutée pour l'analyse d'un cycle courant.
La Figure 9 est une représentation, dans un espace bidimensionnel, des nuages de points correspondant à diverses valeurs de couples de descripteurs relevées, sur un certain nombre de cycles courants, lors de la comparaison avec les deux références correspondant respectivement à une capture ventriculaire et un rythme ventriculaire spontané.

La Figure 10 est une représentation montrant les variations de la distance entre les points de l'espace de la Figure 9 et l'origine de ce même espace, pour des valeurs croissantes du DAV.

La Figure 11 est un organigramme présentant la séquence d'étapes exécutée lors de l'élaboration préalable des deux références utilisées ensuite pour la détermination du degré de fusion d'un cycle courant.

Les Figures 12 et 13 illustrent deux techniques de fenêtrage différentes pour la comparaison du cycle à tester avec la référence en rythme ventriculaire spontané.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply*, *Paradym*, *Intensia*, *Paradym RF* et *Platinium*, produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

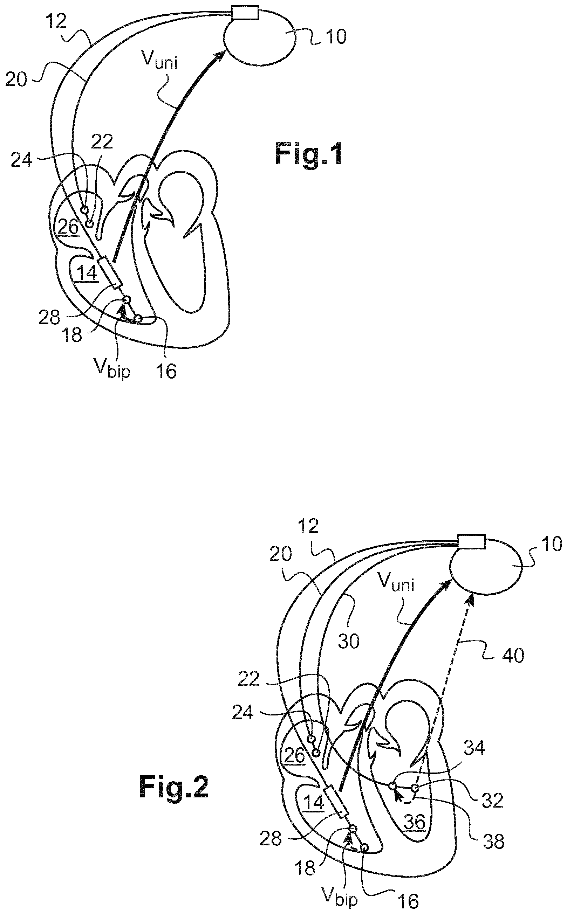

La Figure 1 illustre une configuration typique de stimulation antibradycardique "double chambre", dans laquelle un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18. Une deuxième sonde 20 est pourvue d'électrodes auriculaires de détection distale 22 et proximale 24 situées au niveau de l'oreillette droite 26 pour la détection des signaux dans cette cavité et l'application éventuelle d'une stimulation auriculaire (en variante, ces électrodes auriculaires 22, 24 peuvent être des électrodes flottantes disposées sur la sonde 12 au niveau de l'oreillette).

Dans certaines configurations, la sonde ventriculaire droite 12 peut être également pourvue d'un bobinage (*coil*) ventriculaire 28 formant électrode de défibrillation et permettant aussi de recueillir un signal endocavitaire (ce bobinage pouvant alors être utilisé à la place de l'électrode proximale *ring 18).*

Comme cela sera décrit en détail plus bas, la technique de l'invention met en oeuvre une combinaison de deux signaux d'électrogramme endocavitaire différents recueillis simultanément, en particulier des signaux issus de la même cavité ventriculaire, par exemple du ventricule droit.

La Figure 2 illustre une autre configuration de stimulation à laquelle peut être appliquée l'invention, à savoir une stimulation biventriculaire, notamment afin de rétablir la synchronisation entre les deux ventricules.

A cet effet, le générateur d'impulsions 10, outre les éléments 12 à 18 décrits ci-dessus, est pourvu d'une troisième sonde 30, par exemple une sonde disposée dans le réseau coronaire, comportant une ou plusieurs électrodes 32, 34 disposées au voisinage du ventricule gauche 36 (dans le cas d'une sonde gauche "multi-électrodes", la sonde gauche peut également comporter une ou plusieurs électrodes intermédiaires située(s) dans une position médiane entre les électrodes 32 et 34). Il est ainsi possible d'assurer la stimulation concomitante, ou avec un léger décalage temporel contrôlé (délai interventriculaire DVV), des deux ventricules droit et gauche pour rétablir la synchronisation entre ces deux cavités et améliorer l'hémodynamique générale du patient. Dans le cas d'une sonde gauche multi-électrodes, on peut également appliquer une stimulation multisite à gauche pour traiter un trouble du synchronisme intraventriculaire.

En ce qui concerne spécifiquement la stimulation du ventricule gauche, il est possible d'utiliser une configuration bipolaire (entre les deux électrodes 32 et 34 de la sonde 30) ou unipolaire (entre l'une des électrodes 32 ou 34 et le boitier *can*) du générateur 10. Une sonde quadripolaire peut être également utilisée à ces mêmes fins. Les deux "vecteurs de stimulation" correspondants sont référencés 38 et 40 sur la Figure 2. Ces mêmes vecteurs peuvent être utilisés également pour le recueil d'un signal de dépolarisation ventriculaire gauche. On notera que dans le cas d'une sonde multipolaire il existe un grand nombre de vecteurs bipolaires et unipolaires possibles, définis à partir de chacune des électrodes (on peut également utiliser simultanément plusieurs vecteurs gauches, dans l'hypothèse évoquée plus haut d'une stimulation multisite à gauche).

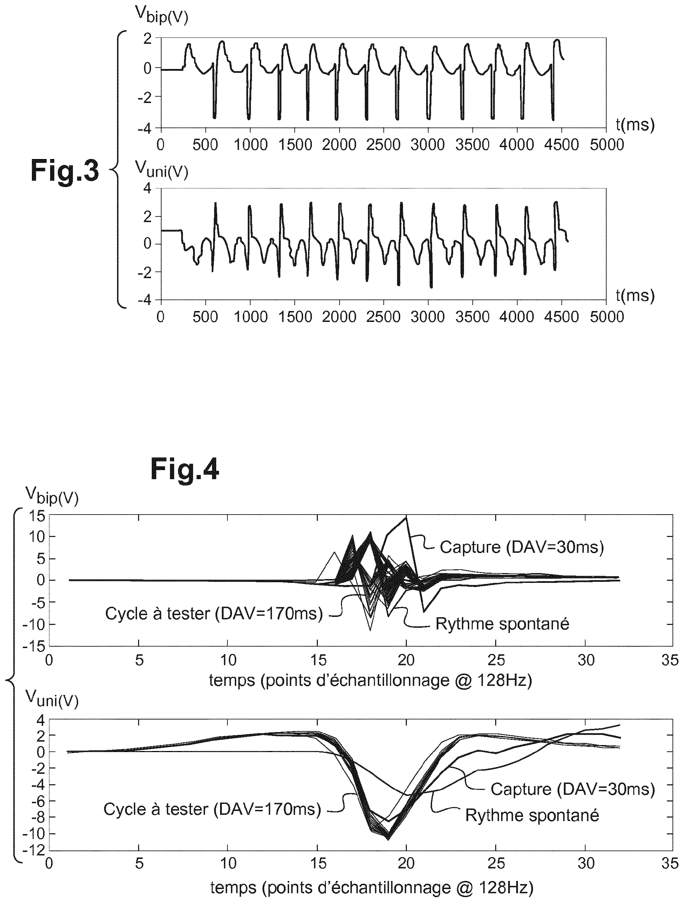

La Figure 3 illustre un exemple de tracés d'EGMs *Vbip* et *Vuni* observés respectivement sur la voie bipolaire ventriculaire et la voie unipolaire ventriculaire de la configuration de la Figure 1 ou de la Figure 2.

Les EGMs recueillis à cet effet dans le ventricule droit peuvent comprendre par exemple :

- une composante ventriculaire droite *Vbip,* dérivée d'un signal EGM *near-field* bipolaire recueilli entre l'électrode distale 16 et l'électrode proximale 18 de la sonde ventriculaire droite 12, et
- une autre composante ventriculaire droite *Vuni,* dérivée d'un signal EGM *far-field* unipolaire recueilli entre le bobinage de défibrillation 36 de la sonde ventriculaire droite 12 et le boitier métallique du générateur 10.

D'autres configurations peuvent être utilisées, à partir de signaux de type *far-field* (par exemple entre l'une des électrodes 16 ou 18 et le boitier 10, ou entre les électrodes 18 et 32) et de type *near-field* (par exemple entre deux électrodes 32 et 34 de la même sonde ventriculaire).

La Figure 4 illustre des exemples de signaux recueillis sur une voie bipolaire (*Vbip*) et sur une voie unipolaire (*Vuni*), pour différentes situations correspondant à une capture complète, à une perte totale de capture laissant apparaitre le rythme ventriculaire spontané, et pour un certain nombre de cycles intermédiaires à tester, avec présence d'une éventuelle fusion plus ou moins marquée, pour des DAV intermédiaires entre les deux situations de capture et de perte de capture.

On peut voir en particulier sur cette figure que l'information n'est pas la même sur les voies bipolaire et unipolaire et que, par exemple, les cycles en fusion (dans cet exemple, avec un DAV à 170 ms) sont beaucoup plus proches morphologiquement du rythme spontané que de la capture sur l'EGM bipolaire, et beaucoup plus proches de la capture sur l'EGM unipolaire.

En ne considérant que l'EGM unipolaire - comme dans la technique décrite par le EP 2 756 865 A1 précité -, la différence est beaucoup moins marquée entre cycles en fusion et cycles de référence avec capture complète ce qui conduirait, si l'on voulait quantifier la fusion, à un pourcentage de fusion (exprimé en pourcentage de capture) beaucoup plus important que la réalité.

Pour ces raisons notamment, la présente invention cherche à optimiser la détection et la quantification de la capture ventriculaire en ajoutant une seconde référence, correspondant à un rythme ventriculaire spontané de manière à identifier doublement le degré de fusion - d'une part par rapport à une capture complète et d'autre part par rapport à un rythme spontané - et également le passage progressif entre fusion et rythme spontané, pour les DAV les plus longs.

La combinaison des deux composantes bipolaire et unipolaire en une caractéristique unique permet de disposer d'une référence contenant de façon plus globale toute l'information disponible issue de l'EGM, ce qui permet de quantifier de façon précise et robuste une éventuelle fusion présente avec le cycle cardiaque courant.

Plus précisément, les deux signaux bipolaire et unipolaire sont combinés en une caractéristique unique de type "boucle cardiaque" ou "vectogramme" (VGM), qui est la représentation dans un espace à deux dimensions de l'un des deux signaux EGM (en ordonnée) par rapport à l'autre (en abscisse). Chaque cycle cardiaque est alors représenté par un vectogramme dans le plan {*Vbip*, *Vuni*} ainsi défini, vectogramme dont la géométrie (forme de la courbe) fait donc abstraction de la dimension temporelle - qui n'intervient que comme un paramètre décrivant la manière dont la courbe est parcourue.

On soulignera que ce "vectogramme" (VGM), qui est obtenu à partir de signaux d'électrogramme (EGM) issu de sondes intracardiaques, ne doit pas être confondu avec le "vectocardiogramme" (VCG) qui est, lui, obtenu à partir de signaux d'électrocardiogramme (ECG) issu d'électrodes externes placées sur le thorax du patient.

La construction d'un VGM et son analyse pour quantifier des données cardiaques sont décrites par exemple dans Milpied et al., "Arrhythmia Discrimination in Implantable Cardioverter Defibrillators using Support Vector Machines applied to a new Représentation of Electrograms," IEEE Transactions on Biomedical Engineering, June 2011, 58(6): 1797-1803. L'analyse d'un VGM a en outre été déjà proposée, comme exposé en introduction, par le EP 2 324 885 A1 (Sorin CRM) pour décider d'une suspicion de fusion afin d'invalider un test de capture.

On notera par ailleurs que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

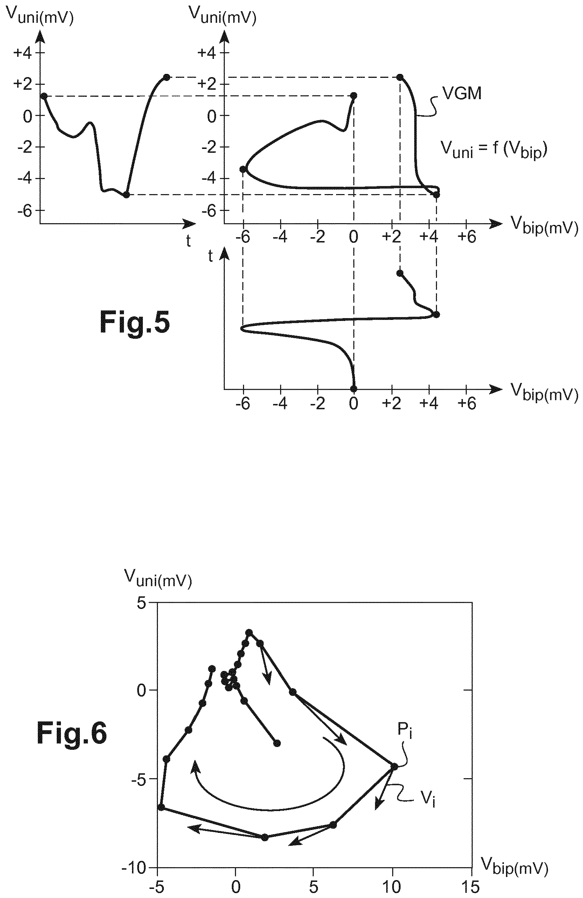

Comme illustré Figure 5, les signaux EGM *Vuni*(*t*) et *Vbip*(*t*) recueillis sont échantillonnés, et les échantillons successifs des deux composantes sont mémorisés puis combinés entre eux pour produire une courbe paramétrique (la caractéristique VGM) du type VGM = (*Vbip*(*t*),*Vuni*(*t*)) ou {x = *Vbip*(*t*), y = *Vuni*(*t*)}.

Cette courbe est une courbe paramétrée par le temps, tracée à partir des variations de l'une des composantes temporelles (*Vuni*) en fonction de l'autre (*Vbip*). Elle constitue un vectogramme (VGM) représentatif du cycle cardiaque à analyser, et sera également désignée "caractéristique 2D paramétrique". Elle présente graphiquement la forme d'une boucle, le temps n'apparaissant plus que dans la manière dont la boucle est parcourue sur la durée du cycle.

En pratique, comme illustré Figure 6, l'échantillonnage produit un VGM en forme de polygone ouvert où chaque sommet correspond à un point d'échantillonnage de la mesure du signal *Vuni* et *Vbip* de l'EGM. Dans l'exemple de la Figure 6, l'échantillonnage est opéré à une fréquence de 128 Hz, ce qui donne environ 11 points de mesure pour un intervalle de temps de 80 ms, qui sont autant de valeurs qui peuvent être mises en mémoire pour être analysées (concrètement, il est possible de conserver en mémoire un plus grand nombre de points, par exemple 60 points, la comparaison entre deux vectogrammes étant en revanche opérée sur un nombre plus restreint de points, typiquement 11 ou 21 points).

On a également représenté sur la Figure 6 l'allure du vecteur vitesse $V_i$ en divers points successifs $P_i$ du VGM pour une fréquence d'échantillonnage de 128 Hz. En un point donné, la vitesse est une donnée vectorielle (la vitesse étant définie par son orientation et sa norme), et le vecteur vitesse peut être calculé en chaque point du VGM à partir d'un filtre discret qui approxime les dérivées premières *Vbip*($t$)/dt et *Vuni*($t$)/dt qui, pour une caractéristique échantillonnée, peuvent être calculées à partir du point précédent et du point suivant sur la courbe.

La caractéristique VGM recueillie est mémorisée sous forme d'une série de paramètres descripteurs basés sur les vecteurs vitesse en chaque point de la courbe et comprenant la norme du vecteur vitesse et l'orientation de ce vecteur vitesse, c'est-à-dire l'angle qu'il fait par rapport à l'axe des abscisses du VGM.

On va maintenant exposer, dans ce contexte, les aspects spécifiques de l'invention.

L'invention propose d'opérer une comparaison morphologique entre, d'une part, le VGM courant (mémorisé sous forme des valeurs des normes et des angles des vecteurs vitesse aux différents points d'échantillonnage) et, d'autre part, deux VGMs de référence (mémorisés sous forme de descripteurs homologues), l'un obtenu en situation de capture complète du ventricule et l'autre obtenu en situation de rythme exclusivement spontané dans le ventricule, c'est-à-dire avec un taux de fusion de 0 % en termes de pourcentage de capture.

La comparaison entre le VGM du cycle courant et ces deux VGMs de référence permet, en cas de détection d'une fusion (c'est-à-dire d'une situation qui n'est ni celle d'une capture complète ni celle d'un rythme spontané ventriculaire), d'évaluer un taux de fusion, permettant ainsi de quantifier par une métrique le décalage temporel plus ou moins important entre l'onde de dépolarisation évoquée consécutive à la stimulation, et l'onde de dépolarisation spontanée liée au rythme naturel.

La comparaison entre le VGM du cycle courant et l'un ou l'autre des VGMs de référence consistera à quantifier leurs ressemblances à partir :

- du coefficient de corrélation C entre les normes des vecteurs vitesse respectifs du VGM courant et du VGM de référence, et
- de la valeur moyenne θ de l'angle que font entre eux les vecteurs vitesse respectifs du VGM courant et du VGM de référence.

On pourra considérer que les courbes se ressemblent si C (qui reflète la corrélation entre les normes des vecteurs vitesse est suffisamment grand, c'est-à-dire proche de l'unité, et θ (qui reflète les écarts angulaires d'orientation) est suffisamment petit, c'est-à-dire proche de zéro. Plus la valeur de C s'éloignera de 1 et plus celle de θ s'éloignera de 0, plus les deux VGMs seront dissemblables.

On notera que l'utilisation des paramètres C et θ pour opérer la comparaison entre deux VGMs n'est pas limitative, et que d'autres paramètres peuvent être utilisés. De la même façon, l'utilisation combinée de C et θ est particulièrement avantageuse, mais il serait également possible d'évaluer la ressemblance des VGMs à partir d'un seul des deux paramètres C ou θ, ou d'un nombre plus important de paramètres, trois paramètres et plus. Les descripteurs C et θ sont cependant préférés, en raison de leur faible sensibilité aux artefacts et de la relative facilité qu'il y a à les calculer.

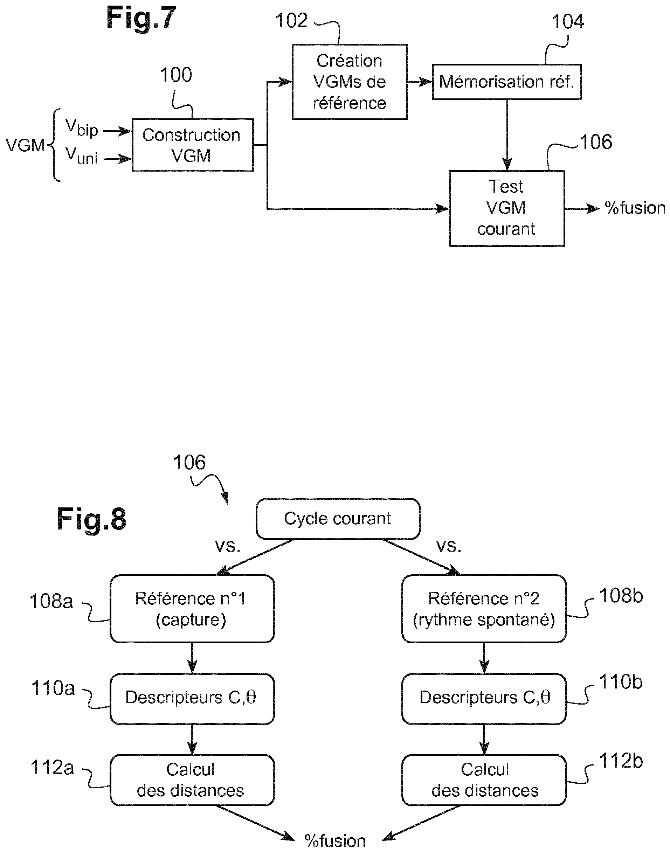

La Figure 7 illustre de façon très générale la manière dont est mis en oeuvre le dispositif de l'invention.

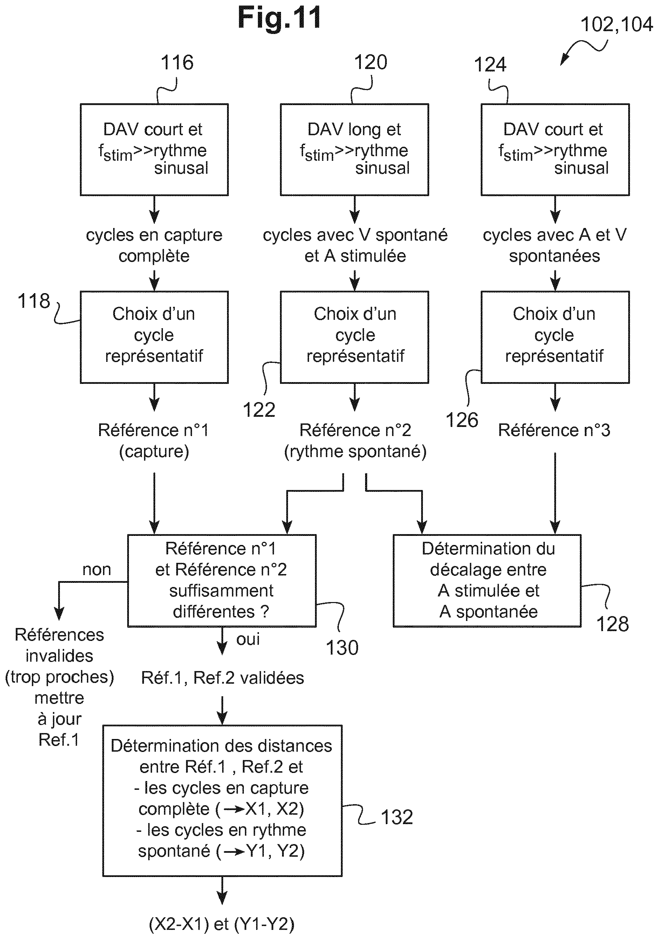

À partir des EGMs bipolaire et unipolaire recueillis, un VGM est construit (bloc 100). Au préalable, deux VGMs de référence sont constitués dans des conditions stables (rythme sinusal lent, de préférence la nuit) (bloc 102, détaillé Figure 11) et conservés en mémoire (bloc 104). Les VGMs courants seront ultérieurement comparés (bloc 106) aux deux VGMs de référence mémorisés, pour produire en sortie une métrique quantifiant un taux de fusion compris entre 0 et 100 %.

Dans la suite, le "taux de fusion" sera exprimé en termes de taux de rythme spontané - c'est-à-dire qu'un taux de fusion de 0 % correspondra à une capture complète et un taux de fusion de 100 % correspondra à une situation de rythme exclusivement spontané - mais l'on pourrait également exprimer ce taux de fusion par rapport à une situation de capture, le taux de fusion variant alors de 100 % à 0 % dans le sens inverse.

La Figure 8 illustre le test du bloc 106, sous forme d'un organigramme simplifié présentant la séquence d'étapes exécutées pour l'analyse d'un cycle courant.

Le VGM du cycle courant est comparé à la fois au VGM de référence correspondant à une situation de capture complète, ci-après "Référence n°1" (bloc 108a) et au VGM correspondant à une situation de rythme spontané ventriculaire, ci-après "Référence n°2" (bloc 108b).

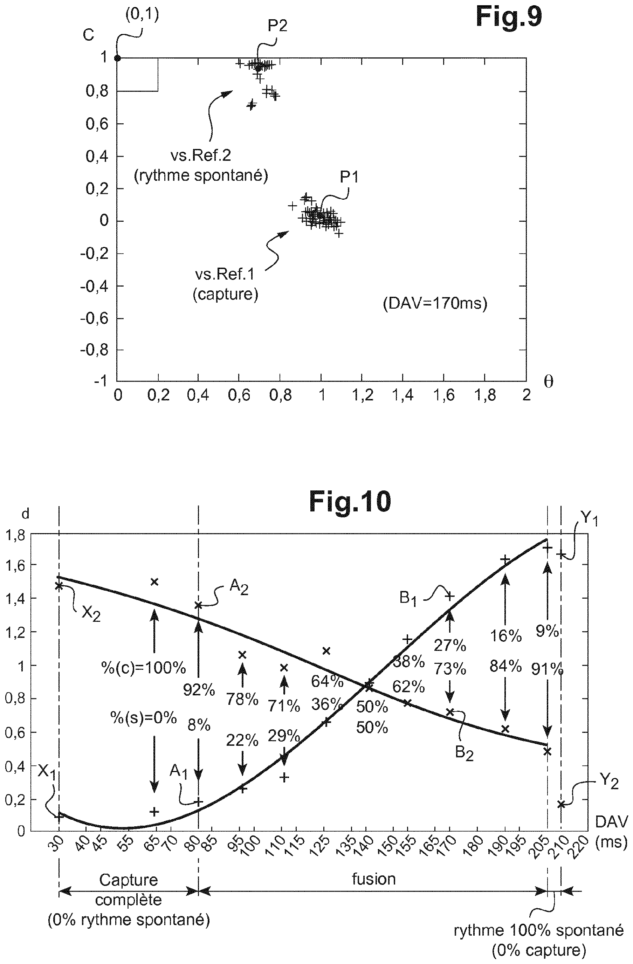

Ces deux comparaisons produisent deux couples respectifs de descripteurs C et θ (blocs 110a et 110b), qui peuvent être représentés dans un espace bidimensionnel illustré Figure 9. L'étape suivante (blocs 112a et 112b) consiste à calculer les distances des divers points [C,θ] de l'espace bidimensionnel correspondant aux descripteurs obtenus par rapport au point d'origine [0,1] de ce même espace, afin de déterminer, à partir de ces distances et de la manière que l'on décrira plus bas, une métrique quantifiant un taux de fusion du cycle courant.

La Figure 9 illustre, dans l'espace bidimensionnel dont les deux dimensions correspondent aux deux descripteurs C et θ, un exemple de répartition des points obtenus pour quelques dizaines de cycles courants avec un même DAV, ajusté en l'espèce à 170 ms. Cet exemple montre deux nuages de points, l'un obtenu par comparaison des cycles courants avec la Référence n°1 (capture complète), l'autre par comparaison avec la Référence n°2 (rythme spontané ventriculaire). Les points P1 et P2 indiquent les barycentres respectifs de ces deux nuages de points.

Le point [0,1] correspond à une situation théorique idéale où le cycle courant serait identique à l'un ou l'autre des cycles de référence, avec un coefficient de corrélation C égal à l'unité et un angle moyen θ nul entre les vecteurs vitesse respectifs des deux VGMs.

La comparaison des VGMs des cycles courants avec chacune des Références 1 et 2 est évaluée :

- soit par comparaison avec des seuils prédéterminés sur C et/ou θ : on considère par exemple que les courbes se ressemblent si C > Seuil 1 et θ < Seuil 2 ;
- soit par une relation entre C et θ : on considère par exemple que les courbes se ressemblent si C > θ ;
- soit, avantageusement, à l'aide de la distance calculée dans le plan [C, θ] des descripteurs entre le point courant et le point [0,1] : plus cette distance est petite, plus les VGMs se ressemblent.

Ainsi, si dans l'exemple illustré on considère la distance entre chacun des points P1 et P2 et le point d'origine [0,1], on constate que les résultats des deux comparaisons respectives montrent que le nuage de points de la comparaison avec la Référence n°2 (rythme spontané) est plus proche de cette origine que le nuage de points de la comparaison avec la Référence n°1 (capture complète), ce qui signifie que les cycles courants sont, en fusion, plus proches d'un rythme ventriculaire spontané que d'une capture complète - donc avec un taux de fusion, exprimé en pourcentage de capture, inférieur à 50 % - sans être toutefois suffisamment proche du point [0,1] pour conclure à un rythme purement spontané.

La Figure 10 est une représentation dans laquelle on a fait varier le DAV entre toutes les valeurs d'ajustement possibles, depuis une valeur minimale DAV = 30 ms produisant en toutes circonstances une capture complète du ventricule, jusqu'à une valeur maximale de DAV suffisamment longue pour laisser s'exprimer le rythme spontané. En ordonnée est portée la distance d entre l'origine [0,1] de l'espace bidimensionnel de la Figure 9 et le point [C,θ] résultant de la comparaison avec la Référence n°1 (points représentés par des "+") ou avec la Référence n°2 (points représentés par des "x").

Pour un DAV minimal DAV = 30 ms en capture ventriculaire complète, on obtient respectivement les repères X1 et X2, et pour un DAV maximal (ici de 210 ms) on obtient les repères respectifs Y1 et Y2.

Sur les figures ont également été indiqués les pourcentages de fusion, exprimés en termes de pourcentages de capture ("%(C)") et en termes de pourcentages de rythme spontané ("%(S)").

Si le cycle courant en fusion est plus proche d'une capture que d'un rythme spontané (partie gauche de la Figure 10, cas des repères A1 et A2), alors le pourcentage de capture est supérieur à 50 % et le pourcentage de rythme spontané est inférieur à 50 %. Inversement, si le cycle courant en fusion est plus proche d'un rythme spontané que d'une capture (partie droite de la Figure 10, cas des repères B1 et B2 correspondant aux points P1 et P2 de la Figure 9), alors le pourcentage de capture est inférieur à 50 % et le pourcentage de rythme spontané supérieur à 50 %. Pour quantifier le degré de fusion, on va considérer l'écart entre les repères X1 et X2 obtenus en capture complète, écart qui est dans cet exemple de l'ordre de X2-X1 = 1,4. De la même façon, l'écart entre les repères Y1 et Y2 en rythme spontané complet est de l'ordre de Y1-Y2 = 1,6. Dans le cas d'un cycle courant en fusion plus proche de la capture que du rythme spontané (repères A1 et A2, où A2 > A1), on devrait toujours avoir A2-A1 < 1,4, et l'on devrait s'éloigner de la capture (A1 > X1) et se rapprocher du rythme spontané (A2 < X2).

Si A2 > A1 et A2-A1 < X2-X1 et si A1 > X1 et A2 < X2 (à une tolérance près, par exemple une tolérance de 0,1 près), alors le pourcentage de capture sera :

$$50 + [50*(A2-A1)/(X2-X1)]$$

Dans le cas contraire, aucun degré de fusion ne sera calculé pour le cycle en question, qui correspond peut-être à une extrasystole et ne suit pas la progression attendue.

On pourra considérer qu'un pourcentage de capture supérieur à un seuil donné, par exemple supérieur à 95 %, correspond à une situation de capture complète, et non de fusion.

Le pourcentage de rythme spontané peut alors être calculé comme étant :

$$100 - \text{le pourcentage de capture.}$$

Si le cycle courant en fusion est plus proche du rythme spontané que de la capture (repères B1 et B2, où B1 > B2), pour calculer le pourcentage de rythme spontané on peut se baser sur la distance entre les repères en rythme spontané Y1 et Y2.

Pour un cycle courant en fusion, on devrait toujours obtenir une valeur B1-B2 < 1,6 et l'on devrait se rapprocher de la capture (B1 < Y1) et s'éloigner du rythme spontané (B2 > Y2).

Si B1 > B2 et B1-B2 < Y1-Y2 et si B1 < Y1 et B2 > Y2 (à une tolérance près, par exemple une tolérance de 0,1 près), alors le pourcentage de rythme spontané sera :

$$50 - [50 * (B1\text{-}B2)/(Y1\text{-}Y2)].$$

Dans le cas contraire, aucun degré de fusion ne sera calculé pour le cycle en question.

On pourra considérer qu'un pourcentage de rythme spontané supérieur à un seuil donné, par exemple supérieur à 95 %, correspond à une situation de rythme spontané et non de fusion.

Le pourcentage de capture peut alors être calculé comme étant :

$$100 - \text{le pourcentage de rythme spontané.}$$

Le taux de fusion final pourra être exprimé soit en termes de taux de capture, soit en termes de taux de rythme spontané, soit par une combinaison de ces deux taux.

En référence aux Figures 11 à 13, on va maintenant décrire la manière d'obtenir les deux références utilisées pour la détermination du taux de fusion.

Ces références sont créées et actualisées de façon périodique et en conditions stables (en particulier la nuit), par exemple une fois par jour ou une fois par semaine.

Pour établir la référence en capture complète (Référence n°1), le DAV est programmé à la valeur la plus courte possible, par exemple DAV = 30 ms, de préférence avec stimulation de l'oreillette à une fréquence de stimulation notablement supérieure au rythme sinusal (bloc 116) de manière à s'éloigner le plus possible des conditions d'apparition d'une fusion éventuelle.

On enregistre dans ces conditions une pluralité de cycles en capture complète, qui sont ensuite comparés de manière à établir ou choisir un cycle représentatif unique (bloc 118). Une première méthode consiste à enregistrer plusieurs VGMs et à les comparer deux à deux, puis vérifier qu'un nombre minimum de VGMs, par exemple deux VGMs se ressemblent. Une autre méthode consiste à rechercher, au fur et à mesure de l'enregistrement des cycles, deux VGMs qui se ressemblent, avec un nombre maximum de VGMs à tester, par exemple cinq VGMs ; si l'on constate une ressemblance suffisante entre les cycles, alors la Référence n°1 est créée et mémorisée, soit en sélectionnant l'un des cycles, soit en moyennant les cycles qui se ressemblent.

Pour la référence en rythme spontané (Référence n°2), un DAV très long est programmé, par exemple DAV = 300 ms, de manière à laisser s'exprimer le rythme spontané avant toute stimulation (bloc 120). Une pluralité de cycles sont ainsi produits de la même façon que pour les cycles en capture complète, et la Référence n°2 est créée pareillement par détermination d'un cycle représentatif unique (bloc 122) après comparaison des différents cycles produits.

Toutefois, pour l'établissement de cette Référence n°2 une fenêtre de mesure plus large est nécessaire, car la partie de l'EGM utilisée pour la comparaison ultérieure des cycles à tester dépendra du DAV desdits cycles à tester. De plus, l'instant de survenue de la dépolarisation n'est pas le même dans le cas d'un événement auriculaire stimulé (évènement A) que dans celui d'une dépolarisation spontanée de l'oreillette (évènement P).

Une première possibilité consiste à établir deux références distinctes en rythme ventriculaire spontané, l'une avec stimulation de l'oreillette et l'autre avec dépolarisation spontanée de l'oreillette (rythme sinusal).

Une autre solution, préférée, consiste à établir une référence unique, par exemple avec dépolarisation stimulée de l'oreillette, et à prévoir une valeur de compensation temporelle ou *offset*, déterminée comme étant la différence des temps de conduction dans le ventricule constatés pour une dépolarisation spontanée de l'oreillette (P) et pour une dépolarisation stimulée de l'oreillette (A) : *offset* = AR-PR.

Ce décalage est établi par une programmation temporaire d'un DAV long avec une fréquence de stimulation très inférieure au rythme sinusal (bloc 124), puis sélection, comme précédemment, d'un cycle représentatif (bloc 126) pour obtenir une référence provisoire (Référence n°3) permettant de déterminer le décalage ci-dessus (bloc 128).

Dans une variante simplifiée, ce décalage peut être établi lors du calcul de la Référence n° 2 en mesurant des intervalles AR, ainsi que des intervalles PR, sans Référence n°3 mais seulement dans les conditions de DAV long et faible fréquence de stimulation (bloc 124), ceci sur quelques cycles (8 cycles par exemple) et en prenant la différence entre la valeur moyenne des intervalles AR et la valeur moyenne des intervalles PR. Une fois établies les deux références Référence n°1 et Référence n°2, une dernière étape consiste à comparer ces deux références en appliquant un DAV fictif de 30 ms et un *offset* si nécessaire (de manière que la condition de l'oreillette et le DAV soient les mêmes). Les deux références sont alors comparées (bloc 130) :

- si la Référence n°1 est bien en capture complète, alors la dépolarisation de la Référence n°2 doit arriver plus tard que celle de la Référence n°1 (pas de superposition dans le temps), la comparaison des courbes devant donc donner une différence importante entre les références. Les références sont alors validées et analysées (bloc 132) de manière à déterminer les repères X1, X2 et Y1, Y2 et les écarts X2-X1 et Y1-Y2 (bloc 132) ;
- si, en revanche, la distance dans l'espace bidimensionnel des descripteurs C et θ entre le point courant et le point [0,1] est inférieure à un seuil donné, par exemple inférieure à l'unité, alors on considère que les références sont trop proches, la Référence n°1 étant sans doute déjà en fusion et non pas en capture complète. Il ne sera donc pas possible de déterminer un degré de fusion correct et la Référence n°1 est alors invalidée comme référence de capture complète.

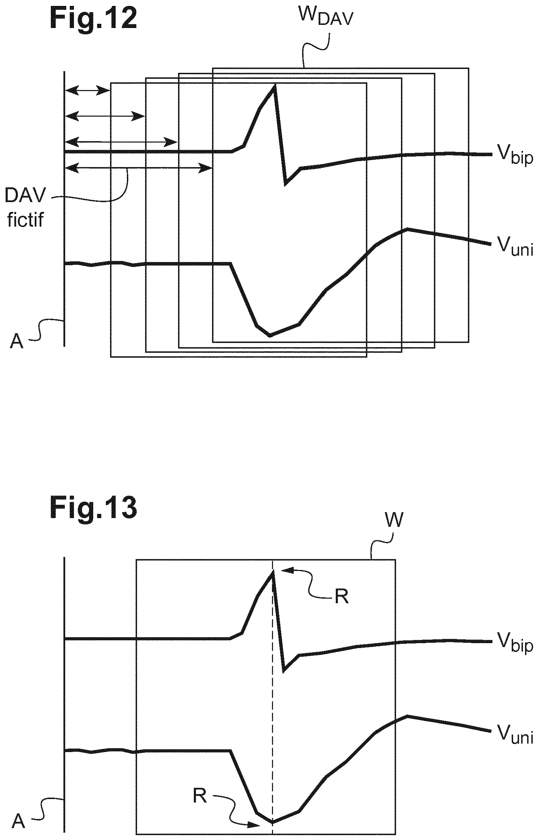

Dans ce dernier cas, il est possible de réitérer le calcul de la Référence n°1 en produisant une pluralité de cycles supplémentaires en mode VVI à une fréquence supérieure à la fréquence de base du patient, par exemple à une fréquence de 100 bpm permettant de récupérer une référence en capture complète. Il suffira alors de comparer à la Référence n°2 tous les cycles ainsi obtenus et de prendre celui qui en diffère le plus tout en étant très proche de la réponse évoquée avec un DAV = 30 ms, cette sélection étant opérée comme précédemment par application de seuils aux valeurs de C et θ. Une autre possibilité consiste à utiliser une Référence n°1 plus ancienne (si elle existe) et la revalider avec la nouvelle Référence n°2. Les Figures 12 et 13 illustrent deux techniques de fenêtrage différentes pour la comparaison du cycle à tester avec la référence en rythme spontané.

En effet, lorsqu'il s'agit de comparer un cycle courant stimulé avec la référence en rythme spontané, un DAV est appliqué au cycle courant alors que les cycles de la référence avaient été obtenus sans DAV.

Comme illustré Figure 12, pour comparer des cycles à tester stimulés avec des DAV variables à la Référence n°2 en rythme spontané, le dispositif applique un "DAV fictif' (fictif, car on est en rythme spontané) ajouté à l'*offset* en cas de non-stimulation de l'oreillette, produisant ainsi un décalage de la fenêtre $W_{DAV}$ d'analyse des EGMs bipolaire et unipolaire. On peut ainsi disposer d'une Référence n°2 unique quelle que soit la valeur du DAV programmable (de 30 à 250 ms typiquement). Par exemple, si l'on veut tester un cycle avec stimulation de l'oreillette et un DAV à 125 ms, on utilisera la Référence n°2 avec un DAV fictif de 125 ms. En effet, le séquencement des EGMs par rapport au marqueur auriculaire A est aussi important que la morphologie des signaux lorsqu'il s'agit de comparer entre eux deux VGMs obtenus à partir de signaux EGM bipolaire et unipolaire respectifs.

La Figure 13 illustre une variante consistant à utiliser, au lieu de la fenêtre mobile de la Figure 12, une fenêtre unique W centrée sur le pic caractéristique (extremum R) du signal EGM *Vbip* ou *Vuni,* indépendamment du type de dépolarisation auriculaire, spontanée ou stimulée. Toutefois, cette solution n'est pas optimale dans la mesure où elle ne tient pas compte du délai de conduction.

## Revendications

**1.** Un dispositif médical implantable actif (10) de stimulation, défibrillation et/ou resynchronisation cardiaque, comportant :

    - des moyens (10, 12, 20 ; 10, 12, 20, 30) de stimulation contrôlée du coeur ;
    - des moyens (10, 12, 20 ; 10, 12, 20, 30) de recueil de signaux de dépolarisation ventriculaire et/ou auriculaire, aptes à recueillir au cours d'un même cycle cardiaque, concurremment sur des voies respectives distinctes, au moins deux signaux différents d'électrogramme endocavitaire, EGM, et en dériver au moins deux composantes temporelles (*Vbip*, *Vuni*) distinctes respectives ; et
    - des moyens d'analyse du cycle cardiaque courant, comprenant :

        • des moyens (100) aptes à combiner les au moins deux composantes temporelles (*Vbip*, *Vuni*) en au moins une caractéristique 2D paramétrique non-temporelle (VGM), paramétrée par le temps, représentative dudit cycle cardiaque, à partir des variations de l'une des composantes temporelles en fonction de l'autre ; et
• des moyens (106) aptes à opérer une comparaison de la caractéristique 2D du cycle courant avec au moins une caractéristique 2D de référence antérieure mémorisée par le dispositif, pour en dériver une valeur d'au moins un descripteur (C, θ) représentatif de la similarité entre la caractéristique 2D du cycle courant et la caractéristique 2D de référence,

le dispositif comprenant en outre des moyens de quantification d'un degré de fusion, comprenant :

- des moyens (108a) pour opérer une première comparaison, de la caractéristique 2D du cycle courant avec une première caractéristique 2D de référence (Référence n°1) en situation de capture complète mémorisée par le dispositif, pour en dériver (110a) une première valeur (A1 ; B1) dudit au moins un descripteur (C, θ) ;
- des moyens (108b) pour opérer une seconde comparaison, de la caractéristique 2D du cycle courant avec une seconde caractéristique 2D de référence (Référence n°2) en situation de rythme spontané ventriculaire mémorisée par le dispositif, pour en dériver (110b) une seconde valeur (A2 ; B2) dudit au moins un descripteur (C, θ) ; et
- des moyens (112a, 112b) pour dériver une métrique (% fusion) quantifiant, par une troisième valeur, un taux de fusion en cas de survenue d'une situation de fusion,
ladite troisième valeur étant fonction à la fois de ladite première valeur et de ladite seconde valeur, et
ladite troisième valeur étant une valeur intermédiaire entre deux extrema respectifs correspondant auxdites première et seconde valeurs obtenues respectivement en situation de capture complète et en situation de rythme spontané ;

dans lequel:

- ledit au moins un descripteur comprend deux descripteurs différents (C, θ), définissant un espace bidimensionnel dont les deux dimensions correspondent aux deux descripteurs ;
- lesdites première et seconde valeurs sont représentées par deux points respectifs (P1 ; P2) dudit espace bidimensionnel ; et
- le dispositif comprend en outre des moyens de calcul de ladite troisième valeur en fonction des distances respectives séparant lesdits deux points (P1 ; P2) d'un repère d'origine ([0,1]) de l'espace bidimensionnel ;

dans lequel l'un des deux descripteurs différents est le coefficient de corrélation (C) entre les normes des vecteurs vitesse respectifs de la caractéristique 2D courante et de la première ou seconde caractéristique 2D de référence ; et dans lequel le taux de fusion, exprimé en termes de pourcentage de capture complète, est donné pour un cycle courant en fusion plus proche de la capture que du rythme spontané par :

$$50 + [50*(A2-A1)/(X2-X1)]$$

et pour un cycle courant en fusion plus proche du rythme spontané que de la capture par :

$$50 - [50 * (B1-B2)/(Y1-Y2)].$$

A2-A1 étant l'écart des distances A1 et A2 séparant, dans ledit espace bidimensionnel, des points représentant respectivement lesdites première et seconde valeurs pour ledit cycle courant en fusion plus proche de la capture que du rythme spontané,
B1-B2 étant l'écart des distances B1 et B2, séparant, dans ledit espace bidimensionnel, des points (P1, P2) représentant respectivement lesdites première et seconde valeurs pour ledit cycle courant en fusion plus proche du rythme spontané que de la capture,
X2-X1 étant l'écart des distances X1 et X2 séparant, dans ledit espace bidimensionnel, des points représentant respectivement lesdites première et seconde valeurs pour des cycles en capture complète, et
Y1-Y2 étant l'écart des distances Y1 et Y2 séparant, dans ledit espace bidimensionnel, des points représentant respectivement lesdites première et seconde valeurs pour des cycles en rythme spontané.

2. Le dispositif de la revendication 1, dans lequel l'autre des deux descripteurs différents est l'angle moyen (θ) entre les vecteurs vitesse respectifs de la caractéristique 2D courante et de la première ou seconde caractéristique 2D de référence.

**3.** Le dispositif de la revendication 1, comprenant en outre des moyens d'établissement de ladite première caractéristique 2D de référence (Référence n°1), comprenant :

- des moyens (116) d'application d'une stimulation avec un délai atrioventriculaire (DAV) court et une fréquence de stimulation supérieure au rythme sinusal spontané, de manière à obtenir une série de cycles cardiaques en situation de capture complète ; et
- des moyens (118) de sélection d'un cycle cardiaque représentatif à partir desdits cycles cardiaques, et de détermination à partir de ce cycle représentatif de ladite première caractéristique 2D de référence (Référence n°1) correspondante.

**4.** Le dispositif de la revendication 1, comprenant en outre des moyens d'établissement de ladite seconde caractéristique 2D de référence (Référence n°2), comprenant :

- des moyens (120) d'application d'une stimulation avec un délai atrioventriculaire (DAV) long et une fréquence de stimulation supérieure au rythme sinusal spontané, de manière à obtenir une série de cycles cardiaques en situation de rythme ventriculaire spontané ; et
- des moyens (120) de sélection d'un cycle cardiaque représentatif à partir desdits cycles cardiaques, et de détermination à partir de ce cycle représentatif de ladite seconde caractéristique 2D de référence (Référence n°2) correspondante.

**5.** Le dispositif des revendications 3 et 4 prises en combinaison, comprenant en outre des moyens de validation desdites caractéristiques 2D de référence, comprenant :

- des moyens (130) pour opérer une comparaison de ladite première caractéristique 2D de référence avec ladite seconde caractéristique 2D de référence, pour en dériver au moins un descripteur (C, $\theta$) représentatif de la similarité entre la première et la seconde caractéristique 2D de référence ; et
- des moyens pour valider ou invalider lesdites caractéristiques 2D de référence selon que ledit au moins un descripteur (C, $\theta$) vérifie(nt) ou non au moins un critère de validation prédéterminé.

**6.** Le dispositif de la revendication 4, comprenant en outre :

- des moyens (124) d'application d'une stimulation avec un délai atrioventriculaire (DAV) long et une fréquence de stimulation inférieure au rythme sinusal spontané, de manière à obtenir une série de cycles cardiaques en situation de dépolarisation spontanée à la fois auriculaire et ventriculaire ;
- des moyens (126) de sélection d'un cycle cardiaque représentatif à partir desdits cycles cardiaques, et de détermination à partir de ce cycle représentatif d'une troisième caractéristique 2D de référence correspondante ; et
- des moyens (128) de détermination, à partir desdites seconde et troisième caractéristiques 2D de référence, d'une valeur de décalage entre événement auriculaire stimulé et événement auriculaire spontané.

**Patentansprüche**

**1.** Aktive implantierbare medizinische Vorrichtung (10) zur Herzstimulation, Defibrillation und/oder Resynchronisation, mit:

Mitteln (10, 12, 20; 10, 12, 20, 30) zur gesteuerten Stimulation des Herzens;
Mitteln (10, 12, 20; 10, 12, 20, 30) zum Erfassen ventrikulärer und/oder atrialer Depolarisationssignale, die während eines selben Herzzyklus gleichzeitig auf jeweils unterschiedlichen Kanälen mindestens zwei verschiedene Signale des endokavitären Elektrogramms, EGM, erfassen und daraus mindestens zwei jeweils unterschiedliche Zeitkomponenten (*Vbip*, *Vuni*) ableiten können; und
Mitteln zum Analysieren des aktuellen Herzzyklus mit:

Mitteln (100), die die mindestens zwei Zeitkomponenten (*Vbip*, *Vuni*) in mindestens einem parametrischen, nicht-zeitlichen 2D-Merkmal (VGM) kombinieren können, das zeitparametriert für den Herzzyklus repräsentativ ist, basierend auf den Schwankungen einer der Zeitkomponenten im Bezug zur anderen; und

Mitteln (106), die einen Vergleich des 2D-Merkmals des aktuellen Zyklus mit mindestens einem früheren, von der Vorrichtung gespeicherten 2D-Referenzmerkmal durchführen können, um daraus einen Wert von mindestens einem Deskriptor (C, θ) abzuleiten, der für die Ähnlichkeit zwischen dem 2D-Merkmal des aktuellen Zyklus und dem 2D-Referenzmerkmal repräsentativ ist,

wobei die Vorrichtung ferner Mittel zum Quantifizieren eines Fusionsgrades umfasst, mit:

Mitteln (108a) zum Durchführen eines ersten Vergleichs des 2D-Merkmals des aktuellen Zyklus mit einem ersten 2D-Referenzmerkmal (Referenz Nr. 1) in einer Situation eines vollständigen, von der Vorrichtung gespeicherten Captures, um daraus einen ersten Wert (A1; B1) dieses mindestens einen Deskriptors (C, θ) abzuleiten (110a);
Mitteln (108b) zum Durchführen eines zweiten Vergleichs des 2D-Merkmals des aktuellen Zyklus mit einem zweiten 2D-Referenzmerkmal (Referenz Nr. 2) in einer Situation eines von der Vorrichtung gespeicherten ventrikulären Eigenrhythmus, um daraus einen zweiten Wert (A2; B2) dieses mindestens einen Deskriptors (C, θ) abzuleiten (110b); und
Mitteln (112a, 112b) zum Ableiten einer Metrik (Fusions-%), die im Fall einer Fusionssituation durch einen dritten Wert eine Fusionsrate quantifiziert,
wobei der dritte Wert sowohl vom ersten Wert als auch vom zweiten Wert abhängig ist und
wobei der dritte Wert ein Zwischenwert zwischen zwei jeweiligen Extremwerten ist, die dem ersten und zweiten Wert entsprechen, die jeweils in einer vollständigen Capture-Situation und in einer Eigenrhythmus-Situation erhalten wurden;

wobei:

der mindestens eine Deskriptor zwei verschiedene Deskriptoren (C, θ) umfasst, die einen zweidimensionalen Raum definieren, dessen zwei Dimensionen den beiden Deskriptoren entsprechen;
der erste und der zweite Wert durch zwei jeweilige Punkte (P1; P2) des zweidimensionalen Raums dargestellt werden; und
die Vorrichtung ferner Mittel umfasst zum Berechnen des dritten Wertes in Abhängigkeit von den jeweiligen Abständen, die die beiden Punkte (P1; P2) von einem Ursprungs-Bezugspunkt ([0,1]) des zweidimensionalen Raums trennen;

wobei einer der beiden verschiedenen Deskriptoren der Korrelationskoeffizient (C) zwischen den Normen der jeweiligen Geschwindigkeitsvektoren des aktuellen 2D-Merkmals und des ersten oder zweiten 2D-Referenzmerkmal ist; und
wobei die Fusionsrate, ausgedrückt als Prozentsatz des vollständigen Capture, für einen aktuellen Fusionszyklus angegeben wird, der dem Capture näher ist als dem Eigenrhythmus, mit:

$$50 + [50*(A2-A1)/(X2-X1)]$$

und für einen aktuellen Fusionszyklus, der dem Eigenrhythmus näher ist als dem Capture, mit:

$$50 - [50 * (B1-B2)/(Y1-Y2)]$$

wobei A2-A1 der Entfernung der Abstände A1 und A2 entspricht, die in dem zweidimensionalen Raum Punkte trennen, die jeweils den ersten und zweiten Wert für den aktuellen Fusionszyklus darstellen, welcher dem Capture näher ist als dem Eigenrhythmus,
wobei B1-B2 der Entfernung der Abstände B1 und B2 entspricht, die in dem zweidimensionalen Raum Punkte (P1, P2) trennen, die jeweils den ersten und zweiten Wert für den aktuellen Fusionszyklus darstellen, welcher dem Eigenrhythmus näher ist als dem Capture,
wobei X2-X1 der Entfernung der Abstände X1 und X2 entspricht, die in dem zweidimensionalen Raum Punkte trennen, die jeweils den ersten und zweiten Wert für vollständige Erfassungszyklen darstellen und
wobei Y2-Y1 der Entfernung der Abstände Y1 und Y2 entspricht, die in dem zweidimensionalen Raum Punkte trennen, die jeweils den ersten und zweiten Wert für Eigenrhythmus-Zyklen darstellen.

**2.** Vorrichtung nach Anspruch 1, bei der der andere der zwei verschiedenen Deskriptoren der Durchschnittswinkel (6) zwischen den jeweiligen Geschwindigkeitsvektoren des aktuellen 2D-Merkmals und des ersten oder des zweiten 2D-Referenzmerkmals ist.

**3.** Vorrichtung nach Anspruch 1, ferner mit Mitteln zum Festlegen des ersten 2D-Referenzmerkmals (Referenz Nr. 1), mit:

Mitteln (116) zum Anlegen einer Stimulation mit einer kurzen atrioventrikulären Verzögerung (AVD) und einer Stimulationsfrequenz, die höher als der eigene Sinusrhythmus ist, um eine Reihe von Herzzyklen in einer vollständigen Capture-Situation zu erhalten; und
Mitteln (118) zum Auswählen eines repräsentativen Herzzyklus aus diesen Herzzyklen und zum Bestimmen aus diesem repräsentativen Zyklus des entsprechenden ersten 2D-Referenzmerkmals (Referenz Nr. 1).

**4.** Vorrichtung nach Anspruch 1, ferner mit Mitteln zum Festlegen des zweiten 2D-Referenzmerkmals (Referenz Nr. 2), mit:

Mitteln (120) zum Anlegen einer Stimulation mit einer langen atrioventrikulären Verzögerung (AVD) und einer Stimulationsfrequenz, die höher als der eigene Sinusrhythmus ist, um eine Reihe von Herzzyklen in einer Situation eines ventrikulären Eigenrhythmus zu erhalten; und
Mitteln (120) zum Auswählen eines repräsentativen Herzzyklus aus diesen Herzzyklen und zum Bestimmen aus diesem repräsentativen Zyklus des entsprechenden zweiten 2D-Referenzmerkmals (Referenz Nr. 2).

**5.** Vorrichtung nach den Ansprüchen 3 und 4 in Kombination, ferner mit Mitteln zum Validieren der 2D-Referenzmerkmale, mit:

Mitteln (130) zum Durchführen eines Vergleichs des ersten 2D-Referenzmerkmals mit dem zweiten 2D-Referenzmerkmal, um mindestens einen Deskriptor (C, θ) abzuleiten, der für die Ähnlichkeit zwischen dem ersten und dem zweiten 2D-Referenzmerkmal repräsentativ ist; und
Mitteln zum Validieren oder Widerrufen der 2D-Referenzmerkmale in Abhängigkeit davon, ob der mindestens eine Deskriptor (C, θ) mindestens ein vorbestimmtes Validierungskriterium überprüft oder nicht.

**6.** Vorrichtung nach Anspruch 4, ferner mit:

Mitteln (124) zum Anlegen einer Stimulation mit einer langen atrioventrikulären Verzögerung (AVD) und einer Stimulationsfrequenz, die niedriger als der eigene Sinusrhythmus ist, um eine Reihe von Herzzyklen in einer Situation von sowohl atrialer als auch ventrikulärer Eigendepolarisation zu erhalten; und
Mitteln (126) zum Auswählen eines repräsentativen Herzzyklus aus diesen Herzzyklen und zum Bestimmen aus diesem repräsentativen Zyklus eines entsprechenden dritten 2D-Referenzmerkmals; und
Mitteln (128) zum Bestimmen eines Ausgleichswerts zwischen dem stimulierten Vorhofereignis und dem eigenen Vorhofereignis aus dem zweiten und dritten 2D-Referenzmerkmal.

**Claims**

**1.** An active implantable medical device (10) for cardiac stimulation, defibrillation and/or resynchronization, the device comprising:

- means (10, 12, 20; 10, 12, 20, 30) for performing controlled stimulation of the heart;
- means (10, 12, 20; 10, 12, 20, 30) for collecting ventricular and/or atrial depolarization signals, which means are suitable for collecting at least two different endocardial electrogram, EGM, signals during the same cardiac cycle, concurrently on respective ones of distinct channels, and for deriving from said signals at least two respective distinct time components (*Vbip*, *Vuni*); and
- means for analyzing the current cardiac cycle, which means comprise:

means (100) suitable for combining the at least two time components (*Vbip*, *Vuni*) into at least one non-time parametric 2D characteristic curve (VGM) parameterized by time and representative of said cardiac cycle, on the basis of the variations of one of the time components as a function of the other time component; and

means (106) suitable for comparing the 2D characteristic curve of the current cycle with at least one prior reference 2D characteristic curve that is stored in a memory by the device so as to derive from the comparison a value for at least one descriptor (C, θ) that is representative of the similarity between the 2D characteristic curve of the current cycle and the reference 2D characteristic curve;

the device further comprises means for quantifying an extent of fusion, which means comprise:

- means (108a) for making a first comparison for comparing the 2D characteristic curve of the current cycle with a first reference 2D characteristic curve (Reference No. 1) in a complete capture situation stored by the device, so as to derive (110a) from the comparison a first value (A1; B1) for said at least one descriptor (C, θ);
- means (108b) for making a second comparison for comparing the 2D characteristic curve of the current cycle with a second reference 2D characteristic curve (Reference No. 2) in a ventricular spontaneous rhythm situation stored by the device, so as to derive (110b) from the comparison a second value (A2; B2) for said at least one descriptor (C, θ); and
- means (112a, 112b) for deriving a metric (percentage of fusion) quantifying, by a third value, a fusion rate in the event a fusion situation occurs; said value being a function both of said first value and of said second value; and said third value being an intermediate value between two respective extrema corresponding to said first and second values obtained respectively in a complete capture situation and in a spontaneous rhythm situation;

wherein:

- said at least one descriptor comprises two different descriptors (C, θ), defining a two-dimensional space, the two dimensions of which correspond to respective ones of the two descriptors;
- said first and second values are represented by two respective points (P1; P2) of said two-dimensional space; and
- the device further comprises means for calculating said third value as a function of the respective distances from said two points (P1; P2) to an origin ([0,1]) of the two-dimensional space;

wherein one of the two different descriptors is the correlation coefficient (C) between the norms of the respective velocity vectors of the current 2D characteristic curve and of the first or second reference 2D characteristic curve; and wherein the fusion rate, expressed as a percentage of complete capture, is given for a current cycle in fusion that is closer to the capture than to the spontaneous rhythm by:

$$50 + [50^*(A2-A1)/(X2-X1)]$$

and for a current cycle in fusion that is closer to the spontaneous rhythm than to the capture by:

$$50 - [50 * (B1-62)/(Y1-Y2)]$$

where A2-A1 is the difference between the distances A1 and A2 that, in said two-dimensional space, separate points representing respective ones of said first and second values for the current cycle in fusion that is closer to the capture than to the spontaneous rhythm;
where B1-B2 is the difference between the distances B1 and B2 that, in said two-dimensional space, separate points (P1, P2) representing respective ones of said first and second values for said current cycle in fusion that is closer to the spontaneous rhythm than to the capture;
where X2-A1 is the difference between the distances X1 and X2 that, in said two-dimensional space, separate points representing respective ones of said first and second values for cycles in complete capture; and
where Y1-Y2 is the difference between the distances Y1 and Y2 that, in said two-dimensional space, separate points representing respective ones of said first and second values for cycles in spontaneous rhythm.

2. The device of claim 1, wherein the other of the two different descriptors is the mean angle (θ) between the respective velocity vectors of the current 2D characteristic curve and of the first or second reference 2D characteristic curve.

3. The device of claim 1, further comprising means for establishing said first reference 2D characteristic curve (Reference No. 1), which means comprise:

- means (116) for applying a stimulation with an atrioventricular delay (AVD) that is short and a stimulation frequency that is greater than the spontaneous sinus rhythm, in such a manner as to obtain a series of cardiac cycles that are in complete capture situations; and

- means (118) for selecting a representative cardiac cycle on the basis of said cardiac cycles, and for determining the corresponding first reference 2D characteristic curve (Reference No. 1) on the basis of that representative cycle.

4. The device of claim 1, further comprising means for establishing said second reference 2D characteristic curve (Reference No. 2), which means comprise:

- means (120) for applying a stimulation with an atrioventricular delay (AVD) that is long and a stimulation frequency that is greater than the spontaneous sinus rhythm, in such a manner as to obtain a series of cardiac cycles that are in spontaneous ventricular rhythm situations; and

- means (120) for selecting a representative cardiac cycle on the basis of said cardiac cycles, and for determining the corresponding second reference 2D characteristic curve (Reference No. 2) on the basis of that representative cycle.

5. The device of claims 3 and 4 taken together, further comprising means for validating said reference 2D characteristic curves, which means comprise:

- means (130) for comparing said first reference 2D characteristic curve with said second reference 2D characteristic curve, so as to derive from the comparison at least one descriptor $(C, \theta)$ that is representative of the similarity between the first and second reference 2D characteristic curve; and

- means for validating or invalidating said reference 2D characteristic curves depending on whether or not said at least one descriptor $(C, \theta)$ satisfies at least one predetermined validation criterion.

6. The device of claim 4, further comprising:

- means (124) for applying a stimulation with an atrioventricular delay (AVD) that is long and a stimulation frequency that is less than the spontaneous sinus rhythm, in such a manner as to obtain a series of cardiac cycles that are in both atrial and ventricular spontaneous depolarization situations;

- means (126) for selecting a representative cardiac cycle on the basis of said cardiac cycles, and for determining a corresponding third reference 2D characteristic curve on the basis of that representative cycle; and

- means (128) for determining a value for offsetting between stimulated atrial event and spontaneous atrial event on the basis of said second and third reference 2D characteristic curves.

**Fig.1**

**Fig.2**

**Fig.3**

**Fig.4**

Fig.5

Fig.6

# Fig.7

```
                                    102 ⌐               104 ⌐
                                  ┌──────────┐        ┌──────────────┐
                                  │ Création │        │ Mémorisation │
                                  │ VGMs de  │───────▶│ réf.         │
                                  │ référence│        └──────────────┘
              100 ⌐               └──────────┘                │
      ┌──Vbip ─▶┌──────────────┐     ▲                        │
 VGM ─┤         │ Construction │─────┘                        ▼
      └──Vuni ─▶│ VGM          │              106 ⌐   ┌──────────┐
                └──────────────┘              │       │ Test     │──▶ %fusion
                                    └─────────────────│ VGM      │
                                                      │ courant  │
                                                      └──────────┘
```

# Fig.8

```
              106 ⌐
                 ┌────────────────────┐
                 │   Cycle courant    │
                 └────────────────────┘
           vs.                       vs.
      ┌─────────────┐           ┌──────────────────┐
108a ─│ Référence n°1│          │ Référence n°2     │─ 108b
      │ (capture)   │           │ (rythme spontané) │
      └─────────────┘           └──────────────────┘
            │                            │
            ▼                            ▼
110a ─┌─────────────────┐     ┌─────────────────┐─ 110b
      │ Descripteurs C,θ│     │ Descripteurs C,θ│
      └─────────────────┘     └─────────────────┘
            │                            │
            ▼                            ▼
112a ─┌─────────────┐         ┌─────────────┐─ 112b
      │ Calcul      │         │ Calcul      │
      │ des distances│        │ des distances│
      └─────────────┘         └─────────────┘
              └──────▶ %fusion ◀──────┘
```

# Fig.9

(0,1)

C

P2

vs.Ref.2
(rythme spontané)

P1

vs.Ref.1
(capture)

(DAV=170ms)

θ

# Fig.10

d

Y₁

X₂

A₂

B₁

%(c)=100%

92%

78%

71%

27%

16%

9%

64%

38%

73%

84%

91%

%(s)=0%

8%

22%

29%

36%

50%
50%

62%

X₁

A₁

B₂

Y₂

DAV
(ms)

Capture
complète
(0% rythme spontané)

fusion

rythme 100% spontané
(0% capture)

# Fig.11

```
    116                  120                  124          102,104

┌──────────────┐    ┌──────────────┐    ┌──────────────┐
│  DAV court et │    │  DAV long et  │    │  DAV court et │
│ fstim>>rythme │    │ fstim>>rythme │    │ fstim>>rythme │
│    sinusal    │    │    sinusal    │    │    sinusal    │
└──────────────┘    └──────────────┘    └──────────────┘
        │                    │                    │
        ▼                    ▼                    ▼
 cycles en capture    cycles avec V spontané  cycles avec A et V
     complète           et A stimulée           spontanées
        │                    │                    │
        ▼                    ▼                    ▼
┌──────────────┐    ┌──────────────┐    ┌──────────────┐
│   Choix d'un  │    │   Choix d'un  │    │   Choix d'un  │
│    cycle      │    │    cycle      │    │    cycle      │
│ représentatif │    │ représentatif │    │ représentatif │
└──────────────┘    └──────────────┘    └──────────────┘
 118                  122                  126
        │                    │                    │
        ▼                    ▼                    ▼
  Référence n°1       Référence n°2        Référence n°3
   (capture)         (rythme spontané)
```

- 116 : DAV court et $f_{stim}$>>rythme sinusal → cycles en capture complète
- 118 : Choix d'un cycle représentatif → Référence n°1 (capture)
- 120 : DAV long et $f_{stim}$>>rythme sinusal → cycles avec V spontané et A stimulée
- 122 : Choix d'un cycle représentatif → Référence n°2 (rythme spontané)
- 124 : DAV court et $f_{stim}$>>rythme sinusal → cycles avec A et V spontanées
- 126 : Choix d'un cycle représentatif → Référence n°3
- 102,104

```
              ┌──────────────────┐         ┌──────────────────┐
     non      │   Référence n°1   │         │  Détermination du │
  ◄───────────│  et Référence n°2 │         │   décalage entre  │
              │   suffisamment    │         │    A stimulée et  │
              │    différentes ?  │         │    A spontanée    │
              └──────────────────┘         └──────────────────┘
                        │ oui                        128
   Références          130
   invalides       Réf.1, Ref.2 validées
  (trop proches)          │
     mettre               ▼
     à jour       ┌──────────────────────────┐
     Ref.1        │ Détermination des distances│
                  │    entre Réf.1 , Ref.2 et  │
                  │  - les cycles en capture   │
                  │    complète (→X1, X2)      │
                  │  - les cycles en rythme    │
                  │    spontané (→Y1, Y2)      │
                  └──────────────────────────┘   132
                            │
                            ▼
                  (X2-X1) et (Y1-Y2)
```

**Fig.12**

**Fig.13**

**EP 3 165 160 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2324885 A1, Sorin CRM

- EP 2756865 A1

**Littérature non-brevet citée dans la description**

- **MILPIED et al.** Arrhythmia Discrimination in Implantable Cardioverter Defibrillators using Support Vector Machines applied to a new Représentation of Electrograms. *IEEE Transactions on Biomedical Engineering,* Juin 2011, vol. 58 (6), 1797-1803